# EUROPEAN PATENT SPECIFICATION

(11) **EP 0 916 736 B1**
(45) Date of publication and mention of the grant of the patent: **16.11.2005**
(21) Application number: 97924322.7
(22) Date of filing: 05.06.1997
(51) Int. Cl.: C12P 33/06

(54) **BIOLOGICAL PROCESS FOR PREPARING 25-HYDROXYLATED STEROIDS**
BIOLOGISCHES VERFAHREN ZUR HERSTELLUNG VON 25-HYDROXYLIERTEN STEROIDEN
PROCEDE BIOLOGIQUE SERVANT A PREPARER DES STEROIDES 25-HYDROXYLES

(30) Priority: 27.06.1996 JP 16696896
(43) Date of publication of application: 19.05.1999
(73) Proprietor: MERCIAN CORPORATION, Chuo-ku, Tokyo 104-8305 (JP); CHUGAI SEIYAKU KABUSHIKI KAISHA, Tokyo 115 (JP)
(72) Inventor: TAKEDA, Koji, Iwata-shi, Shizuoka-ken 438 (JP); TERASAWA, Tadashi, Fujisawa-shi, Kanagawa-ken 251 (JP); DOBASHI, Kazuyuki, Hadano-shi, Kanagawa-ken 257 (JP); YOSHIOKA, Takeo, Ayase-shi Kanagawa-ken 252 (JP)
(74) Representative: Paget, Hugh Charles Edward
(86) International application number: PCT/JP1997/001909
(87) International publication number: WO 1997/049829

(56) References cited:
- EP-A- 0 298 757
- EP-A- 0 653 490
- WO-A-97/08336
- JP-A- 2 231 089
- JP-A- 4 356 190
- JP-A- 7 123 997
- JP-A- 7 241 197
- KOJI TAKEDA ET AL.: "Application of cyclodextrin to microbial transformation of Vitamin D3 to 25-hydroxyvitamin D3 and 1alpha,25-dihydroxyvitamin D3" JOURNAL OF FERMENTATION AND BIOENGINEERING, vol. 78, no. 5, 1994, pages 380-382, XP001053545
- JOJI SASAKI ET AL.: "Transformation of vitamin D3 to 1alpha,25-dihydroxyvitamin D3 via 25-hydroxyvitamin D3 using Amycolata sp. strains" APPLIED MICROBIOLOGY AND BIOTECHNOLOGY, vol. 38, no. 2, 1992, pages 152-157, XP001053544
- J. CLIN. INVEST., Vol. 57, No. 4, (1976), S. SHEFER et al., "A25-Hydroxylation Pathway of Cholic Acid Biosynthesis in Man and Rat", p. 897-903, XP002908925.

## Description

### Technical Field

The present invention relates to a process for biologically hydroxylating the 25-position of steroids other than cholesterol.

### Background of the Invention

For the biological hydroxylation of steroids at the 25-position, there is already known a process wherein microorganisms of the genus Streptomyces capable of hydroxylating steroids at the 25-position thereof are used [Japanese Patent Unexamined Published Application (hereinafter referred to as "J. P. KOKAI") No. Hei 7-123997]. Concretely, Streptomyces sp. HB-103 is mentioned therein as the microorganisms of the genus Streptomyces. With this kind of microorganisms, steroids having complicated structures can be efficiently, easily and directly hydroxylated at the 25-position. Amycolata autotrophica has been used to convert vitamin D₃ to 1α,25 dihydroxyvitamin D₃ by hydroxylation at the 25-position. (Journal of Fermentation and Bioengineering, vol 78, no. 5, 1994, 380-382; Applied Microbiology and Biotechnology, vol 38, no. 2, 1992, 152-157; JP 02 231089).

### Disclosure of the Invention

The object of the present invention is to provide a biological process for hydroxylating steroids, other than cholesterol, at the 25-position thereof with microorganisms of a genus other than the genus Streptomyces.

The present invention has been completed on the basis of a finding that among various microorganisms, those of the genus Amycolata or Sphingomonas are capable of hydroxylating steroids at the 25-position thereof.

Namely, the present invention provides a biological process for producing steroids hydroxylated at the 25-position thereof according to claim 1 herein.

### Best Mode for Carrying Out the Invention

The steroids to be hydroxylated in the present invention are compounds of the following general formula (I): 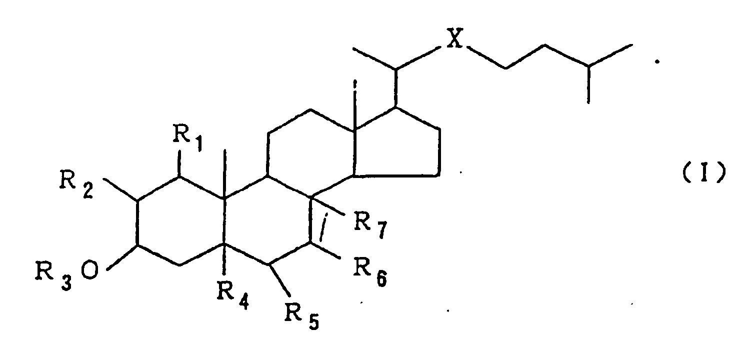 wherein R₁ represents a hydrogen atom or a hydroxyl group which may be optionally protected; R₂ represents a hydrogen atom or a hydroxy-lower alkoxyl group in which the hydroxyl group may be optionally protected, or R₁ and R₂ together form a double bond or epoxy ring, R₉ represents a hydrogen atom or a protecting group; R₄, R₅, R₆ and R₇ each represent a hydrogen atom, one or both couples of R₄ and R₅, and R₆ and R₇ form a double bond or, alternatively, R₅ and R₆ together form a double bond and R₄ and R₇ are bonded to a dienophile capable of protecting the conjugated double bond; and X represents CH₂ or an oxygen atom, with the proviso that when X is CH₂, a compound wherein R₁, R₂, R₈ and R₇ are hydrogen atom and R₄ and R₅ together form a double bond is excluded.

The protecting groups in the above formula are, for example, acyl groups such as acetyl, pivaloyl, methoxycarbonyl, benzyloxycarbonyl and p-toluenesulfonyl groups; alkyl groups which may be optionally substituted such as methyl and methoxymethyl groups; and substituted silyl groups such as trimethylsilyl, triethylsilyl and t-butyldimethylsilyl groups. Among them, trimethylsilyl, triethylsilyl and t-butyldimethylsilyl groups are preferred.

As the dienophiles capable of protecting the conjugated double bonds, compounds of the following general formula (II) are used: 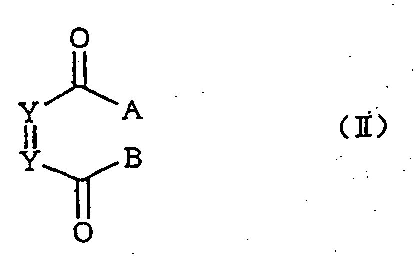 wherein A and B may be the same or different and each represent an alkoxyl group having 1 to 4 carbon atoms, or A and B together form a phenylimino group or o-phenylene group, and Y represents a nitrogen atom or a methine group (=CH-). Among them, 4-phenyl-1,2,4-triazoline-3,5-dion, diethyl maleate, etc. are preferred.

The steroids to be hydroxylated in the present invention include 1α,3β-dihydroxy-5,7-cholestadiene, 2β-(3-hydroxypropyloxy)-1α,3β -dihydroxy-5,7-cholestadiene,5α,8α-(3,5-dioxo-4-phenyl-1,2,4-triazolizino)-1,6-cholestadiene-3β-ol, 3β-hydroxy-1,5,7-cholestatriene, 1α,2α -epoxy-5α, 8α-(3,5-dioxo-4-phenyl-1,2,4-triazolizino)-6-cholesten-3β-ol, 1α,2α-epoxy-3β-hydroxy-5,7-cholestadiene, 20(s)-(3-methylbutyloxy)pregna-5,7-diene-1α,3β-diol, 20(S)-(3-methylbutyloxy)-pregna-5-ene-1α,3β-diol and the like.

According to the process of the present invention, the steroids can be hydroxylated at the 25-position irrespective of the positions or the number of hydroxyl groups in the steroids and, further, they can be hydroxylated at the 25-position irrespective of the positions or the number of double bonds.

The microorganisms of the genus Amycolata capable of hydroxylating the steroids at the 25-position thereof and usable in the present invention include, for example, those of strains of Amycolata saturnea A-1246, Amycolata saturnea FERM BP-2307 and Amycolata autotrophica ATCC 33796. However, microorganisms of any strain of the genus Amycolata are usable so far as they are capable of hydroxylating the steroids at the 25-position thereof. The microorganisms of the genus Sphingomonas include those of, for example, Sphingomonas parapaucimobilis IFO 15100. Microorganisms of any strain of the genus Sphingomonas are usable so far as they are capable of hydroxylating the steroids at the 25-position thereof.

Among them, the strains Amycolata autotrophica ATCC 33796 and Amycolata saturnea A-1246 are preferred. Amycolata saturnea A-1246, which was separated from the soil by the inventors, has the following bacteriological properties:
(1) Morphological properties:
   The vegetative hyphae grow well and irregularly branch in a synthetic agar medium or a natural agar medium. No septum is found. The spores are well formed in a glycerol/asparagine agar medium, a starch/inorganic salt agar medium or the like. It is found in the microscopic observation that the sporulation hyphae are simply branched, and the spores are linearly formed. Usually at least three spore linkages are observed, and the chains are long and have the smooth surface in the latter period of the culture. The spores are cylindrical and the size thereof is 0.5 to 0.8 × 2.5 to 4.3 µm. The sclerotium, sprangium and flagellate spore were not observed.
(2) Growth in various media (30°C):
   (2-1) Sucrose/nitrate agar medium:
      The growth of the microorganisms on the medium is moderate. The color tone of the back surface of the colony is light brown. The formation of aerial hyphae is medium and they are yellowish white. No soluble pigment is formed.
   (2-2) Glucose/asparagine agar medium:
      The growth of the microorganisms on the medium is relatively poor. The color tone of the back surface of the colony is yellowish white. The formation of aerial hyphae is medium and they are white. No soluble pigment is formed.
   (2-3) Glycerol/asparagine agar medium:
      The growth of the microorganisms on the medium is good. The color tone of the back surface of the colony is light yellow. The formation of aerial hyphae is good and they are white. No soluble pigment is formed.
   (2-4) Starch/inorganic salt agar medium:
      The growth of the microorganisms on the medium is moderate. The color tone of the back surface of the colony is yellowish white. The formation of aerial hyphae is good and they are white. No soluble pigment is formed.
   (2-5) Tyrosine agar medium:
      The growth of the microorganisms on the medium is moderate. The color tone of the back surface of the colony is reddish brown. The formation of aerial hyphae is good and they are yellowish white. A soluble light reddish brown pigment is formed.
   (2-6) Nutrient agar medium:
      The growth of the microorganisms on the medium is good. The color tone of the back surface of the colony is light yellow. The formation of aerial hyphae is good and they are white. No soluble pigment is formed.
   (2-7) Yeast/malt agar medium:
      The growth of the microorganisms on the medium is good. The color tone of the back surface of the colony is light yellow. The formation of aerial hyphae is good and they are white. No soluble pigment is formed.
   (2-8) Oat meal agar medium:
      The growth of the microorganisms on the medium is moderate. The color tone of the back surface of the colony is yellowish white. The formation of aerial hyphae is relatively poor and they are white. No soluble pigment is formed.
   (2-9) Peptone/yeast/iron agar medium:
      The growth of the microorganisms on the medium is moderate. The color tone of the back surface of the colony is light brown. The formation of aerial hyphae is moderate and they are yellowish white. No soluble pigment is formed.
(3) Physiological properties:
   (3-1) Growth temperature range: When the nutrient agar medium is used, a good growth is observed in the temperature range of 20 to 30 °C . The microorganisms do not grow at 10 °C or below, or at 40 °C or above.
   (3-2) Aerobe or anaerob: aerobe
   (3-3) Gelatin liquefaction: positive
   (3-4) Starch hydrolysis: negative
   (3-5) Coagulation and peptonization of skim milk powder: both negative
   (3-6) Formation of melanin-like pigment: negative
   (3-7) Nitrate reductivity: negative.
(4) Utilization of carbon sources: Various carbon sources were each applied to Pridham-Gottlieb's agar medium and the growth of microorganisms was observed. Carbon sources such as D-glucose, sucrose, D-xylose, inositol, D-mannit and D-fructose were usable, while L-arabinose, L-rhamnose and raffinose were unusable.
(5) Cell wall components were examined by using decomposition products of the whole cells to find that the cell walls belong to type III cell walls according to Lechevalier classification [International Jurnal of Systematic Bacteriology, vol. 20, pp. 435 to 443 (1970)]. Mycolic acid was not contained therein.

It is apparent from the above-described bacteriological properties that this strain belongs to actinomycetes. By comparing these properties with those of known microorganisms reported in Internatioonal Journal of Systematic Bacteriology, Vol. 36, pp. 29 to 37 (1986), this strain was substantially identified with Amycolata saturnea. From those results, this strain was judged to belong to Amycolata saturnea, and named "Amycolata saturnea" A-1246. This strain was deposited with the National Institute of Bioscience and Human-Technology of the Agency of Industrial Science and Technology (1-3, Higashi 1-chome, Tsukuba City, Ibaraki Pre f., Japan) to be preserved under FERM BP-5544 on August 7, 1995.

The biological process of the present invention for hydroxylating steroids (other than cholesterol) at the 25-position comprises hydroxylating steroids (other than cholesterol) as the substrate under aerobic conditions in a solution containing microorganisms of the genus Amycolata or Sphingomonas. The cells of the microorganism necessitated for the reaction are produced by inoculating the above-described strain into a nutrient source-containing medium and culturing the microorganisms under aerobic conditions. As a rule, the microorganisms are cultured by an ordinary culture method. Usually, they are preferably cultured under aerobic conditions by, for example, the shaking culture method or spinner culture method under aeration.

As for the medium, any medium is usable so far as it contains nutrient sources for the microorganisms belonging to the genus Amycolata or Sphigomoonas. Various synthetic, semi-synthetic and natural media are usable. As for the composition of the medium, carbon sources such as glucose, maltose, xylose, fructose and sucrose are usable either alone or in combination of them. As the nitrogen sources, organic nitrogen sources such as peptone, meat extract, soybean powder, casein, amino acids, yeast extract and urea; and inorganic nitrogen sources such as sodium nitrate and ammonium sulfate are usable either alone or in combination of them. In addition, salts such as sodium chloride, potassium chloride, calcium carbonate, magnesium sulfate, sodium phosphate, potassium phosphate and cobalt chloride, heavy metal salts and vitamines can also be added if necessary. When the foaming is marked in the course of the culture, a defoaming agent selected from among well known defoaming agents can be properly added to the medium.

The culture conditions can be suitably selected so as to grow the microorganisms of this strain well. Usually, the microorganisms are cultured at a pH of 6 to 7.5 at 28 to 30 °C for about 2 to 8 days. The above-described culture conditions can be suitably altered depending on the kind and characteristics of the microorganisms used and external conditions.

A steroid is added to the reaction solution containing the microorganism cells thus obtained to produce a corresponding steroid having a hydroxyl group at the 25-position. Namely, the culture fluid containing the microorganism cells is directly used or, alternatively, cells are separated from the culture fluid by the centrifugation or filtration after the completion of the culture, and the obtained cells are suspended in a solution to obtain a suspension to be used. The solutions in which the cells can be suspended include the above-described media, and buffer solutions such as tris(hydroxymethyl) aminomethane-acetic acid, tris(hydroxymethyl)aminomethane-hydrochloric acid, sodium succinate, sodium citrate, sodium phosphate and potassium phosphate which are to be used either alone or in the form of a mixture of them. The pH of the buffer solution is preferably 7.0 to 8.5.

The steroid to be used as the substrate is in the form of the powder itself or a solution thereof in a water-soluble organic solvent such as ethanol. The powder or the solution is added to the reaction solution containing the microorganism cells. The amount of the powder or solution is preferably 0.15 to 0.60 mg per ml of the reaction solution. When the amount thereof is larger than 0.60 mg/ml, the conversion velocity is lowered unfavorably. After the addition of the substrate, the shaking or spinning under aeration is conducted at 27 to 31°C for one to three days, preferably about one day, to conduct the reaction under aerobic conditions, thereby producing the steroid having hydroxyl group at the 25-position thereof.

As described above, steroids having a hydroxyl group at the 25-position thereof can be obtained from steroids by using microorganisms of the genus Amycolata or Sphingomonas. Further, the conversion of the steroids into those having a hydroxyl group at the 25-position thereof can be remarkably increased by introducing a cyclodextrin or a cyclodextrin derivative together with the steroid into the microorganism reaction solution.

The cyclodextrins used in the present invention include β-cyclodextrin and γ-cyclodextrin. The cyclodextrin derivatives include, for example, hydroxypropyl-β-cyclodextrin, maltosyl-β-cyclodextrin, glycosyl-β-cyclodextrin and methylated cyclodextrins. Among them, the methylated cyclodextrins are preferred. The term "methylated cyclodextrins" herein indicates compounds obtained by replacing the hydrogen atom of the hydroxyl group at the 2-, 3- and/or 6-position of cyclodextrin with a methyl group. They include hexakis-(2,6-O-dimethyl)-α-cyclodextrin which is derived from α-cyclodextrin and completely methylated at the 2- and 6-positions, heptakis-(2,6-O-dimethyl)-β-cyclodextrin which is derived from β-cyclodextrin and octakis-(2,6-O-dimethyl)-γ-cyclodextrin which is derived from γ-cyclodextrin; hexakis-(2,3,6-O-trimethyl)-α-cyclodextrin which is derived from α-cyclodextrin and completely methylated at the 2-, 3- and 6-positions, heptakis-(2,3,6-O-trimethyl)-β-cyclodextrin which is derived from β-cyclodextrin and octakis-(2,3,6-O-trimethyl)-γ-cyclodextrin which is derived from γ-cyclodextrin; and partially methylated cyclodextrins (hereinafter referred to as "PMCD") obtained by partially methylating six, seven or eight hydroxyl groups at the 2-, 3- and 6-positions. The methylation rate is preferably 50 to 70 %, most preferably about 61 %. One or more members of the above-described methylated cyclodextrins are selectively used in the present invention. Methylated cyclodextrins derived from β-cyclodextrin are particularly preferred.

The amount of the cyclodextrins or derivatives thereof is preferably 0.1 to 10 mg, more preferably 0.5 to 5 mg, per ml of the reaction solution. When the amount of the cyclodextrins or derivatives thereof is less than 0.1 mg per ml of the reaction solution, the effect of improving the conversion into the steroids having hydroxyl group at the 25-position is insufficient and, on the contrary, when it exceeds 10 mg, the conversion reaction rate is lowered, and the foaming of the reaction solution becomes serious to make the continuation of the microbial reaction difficult.

It is effective to use cyclodextrins together with a surfactant in the present invention. The surfactants are preferably nonionic surfactants such as polyoxyethylene / sorbitan fatty acid esters [such as Tween 80 (TM) (a product of Sigma Chemical Company), sorbitan fatty acid esters [such as Span 85 (TM) (a product of Sigma Chemical Company)], polyoxyethylene ethers [such as Brij 96 (TM) (a product of Sigma Chemical Company)], Triton X-100 (TM) (a product of Sigma Chemical Company), nonylphenol [such as Nonipole 45 (TM) (a product of Sanyo Chemical Industries, Ltd.) and ethylene oxide / propylene oxide block copolymers [such as Pluronic L-61 (TM) (a product of Asahi Denka Kogyo K.K.); and anionic surfactants such as Dilex (TM) a product of Nippon Oils and Fats Co., Ltd.) and Trax (TM) (a product of Nippon Oils and Fats Co., Ltd.). The amount of the nonionic surfactant used is preferably about 0.1 to 0.5 %

The steroid having hydroxyl group at the 25-position produced by the reaction can be isolated by any of various known methods or a combination of them. The isolation and purification can be conducted by, for example, the extraction with a solvent such as ethyl acetate or n-butanol; column chromatography with silica gel or the like; thin-layer chromatography, liquid-liquid partition chromatography, preparative high-performance liquid chromatography wherein reversed phase column is used; or column chromatography wherein a synthetic adsorbent resin is used. These methods can be employed either solely or in a suitable combination of them, or they can be repeated if necessary.

The following Examples will further illustrate the present invention, which by no means limit the invention, wherein percentages are given by weight unless otherwise stated.

### Example 1

100 ml of a seed culture medium (hereinafter referred to as "medium A") comprising 1.5 % of glucose, 1.5 % of Bacto® Soytone (a product of Difco), 0.5 % of corn steep liquor (Nihon Shokuhin Kako Co., Ltd.), 0.4 % of sodium chloride, 0.2 % of calcium carbonate (pH 7.0) and the balance of water was fed into a 500 ml Erlenmeyer flask and sterilized by heating at 120°C for 20 minutes. 2 ml of frozen yeast culture of Amycolata autotrophica ATCC 33796 was inoculated into the medium. After the shaking culture at 220 rpm (amplitude: 70 mm) at 28°C for two days, the seed culture fluid was obtained.

Then, 50 ml of a conversion culture medium (hereinafter referred to as "medium B") comprising 2.0 % of glucose, 0.2 % of yeast extract (Oriental Yeast Co. Ltd.), 0.5 % of peptone (Kyokuto Pharmaceutical IND., Co., Ltd.), 1.0 % of soybean powder (Esusan Meat; Ajinomoto Co., Lt d.), 0.5 % of corn step liquor, 0.04 % of potassium secondary phosphate, 0.04 % of sodium chloride, 0.2 % of calcium carbonate (pH 7.4) and the balance of water was fed into a 250 ml Erlenmeyer flask and sterilized by heating at 120°C for 20 minutes. 2 ml of the seed culture fluid prepared as described above was inoculated into the medium. After the culture with a rotary shaker at 28°C for two days, the fluid were poured in test tubes each in an amount of 3 ml.

Partially methylated β-cyclodextrin (methylation rate: 55.8 %) was added to each 3 ml culture fluid to obtain a concentration of 0, 0.5 or 1.0 % by weight. 250 µg/ml of the following steroid a, b, c or d in the form of a solution in ethanol was added to the resultant mixture. The reaction was conducted in a rotary shaker at 30 °C for 17 hours.
steroid a: 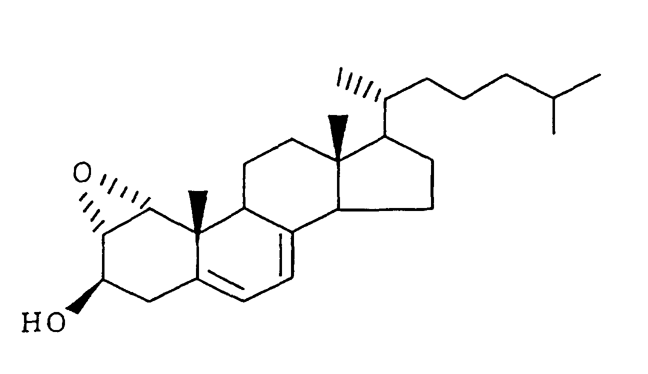 steroid b: 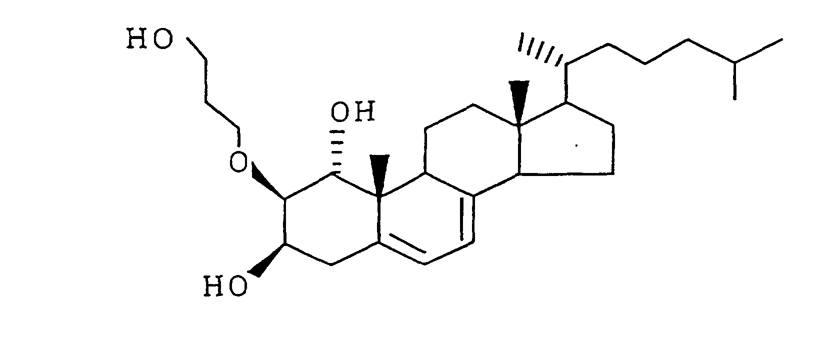 steroid c: 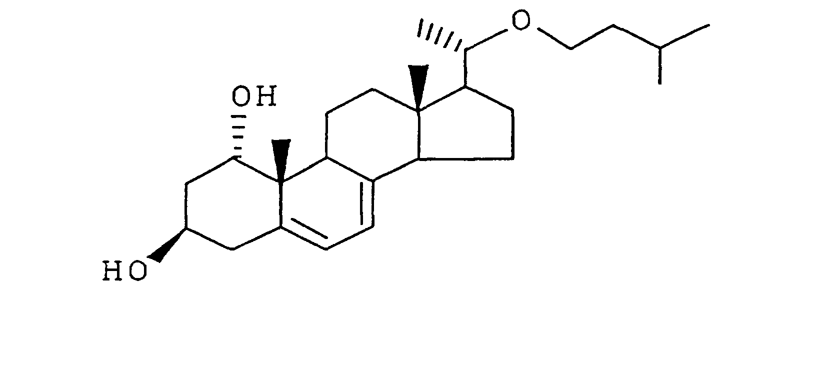 steroid d:

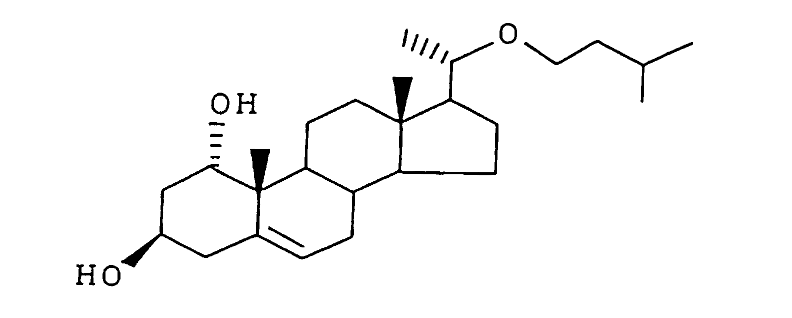

After the completion of the reaction, 1 ml of the obtained culture fluid was fed into a centrifugal precipitation tube having a ground stopper. 9 ml of methanol was added to the fluid, the bottle was tightly stopped and they were mixed for 15 minutes. The liquid mixture was centrifuged at 3,000 rpm for 10 minutes, and the supernatant liquid was analyzed by HPLC. The analysis was conducted by using an HPLC apparatus of L-6000 System (a product of Hitachi, Ltd.), a column of YMC A 503CN (inner diameter: 4.6 mm, the whole length: 250 mm; a product of Yamamura Chemistry) and an eluent comprising acetonitrile and water (55:45) at a flow rate of 1.0 ml/min and at a column temperature of 40 °C. The detection was conducted according to the UV absorption at 205 or 265 nm. The quantitative analysis was conducted by the comparison with the areametric values of chemically synthesized standard steroids, a, b, c or d hydrolyzed at the 25 position.

**Table 1**

| [Analytical values obtained after 17 hour conversion reaction] (unit: µg/ml) | | | | |
|---|---|---|---|---|
| | | PMCD 0% | PMCD 0.5% | PMCD 1.0% |
| Steroid a | Substrate | 127 | 116 | 7.16 |
| | 25-OH-steroid | 3.77 | 82.6 | 169 |
| Steroid b | Substrate | 110 | 50.2 | 4.57 |
| | 25-OH-steroid | 4.05 | 89.8 | 158 |
| Steroid c | Substrate | 0 | 0 | 0 |
| | 25-OH-steroid | 5.85 | 53.7 | 41.8 |
| Steroid d | Substrate | 0 | 0 | 0 |
| | 25-OH-steroid | 6.90 | 23.4 | 83.6 |

It is apparent from the above results that according to the present invention, steroids each having hydroxyl group at the 25-position can be efficiently obtained from the steroids and that the rate of the hydroxylation at the 25-position of each steroid is increased in the presence of methylated cyclodextrin.

### Example 2

The reaction was conducted using steroid a as the substrate for six hours in the same manner as that of Example 1 except that the concentration of PMCD was varied. The quantity of the steroid having hydroxyl group at the 25 position was determined. The results are shown in Table 2.

**Table 2**

| [Quantity of 25-hydroxylated steroid after 6 hour reaction] | | | | | |
|---|---|---|---|---|---|
| PMCD conc. (%) | 0 | 0.25 | 0.50 | 1.0 | 2.0 |
| 25-OH-steroid (µg/ml) | 6.7 | 25.1 | 44.5 | 110 | 23.2 |

It is apparent from the above results that the optimum concentration of the methylated cyclodextrin is 1 %.

### Example 3

The reaction was conducted using steroid a as the substrate for six hours in the same manner as that of Example 1 except that the concentration of PMCD was fixed at 1 % and the concentration of the substrate was varied. The quantity of the steroid having hydroxyl group at the 25 position was determined. The results are shown in Table 3.

**Table 3**

| [Quantity of 25-hydroxylated steroid after 6 hour reaction] (unit: µg/ml) | | | | | |
|---|---|---|---|---|---|
| Substrate conc. | 65 | 125 | 250 | 500 | 1000 |
| 25-OH-steroid | 29.8 | 57.5 | 90.4 | 10.7 | 15.7 |

It is apparent from the above results that the optimum concentration of the steroid is 250 µg/ml.

### Example 4

The reaction was conducted using steroid a as the substrate for 6 hours or 24 hours in the same manner as that of Example 1 except that 1.0 % of various cyclodextrins and derivatives thereof were used and that 0 or 0.2 % of Tween 80 (TM) was used. The quantity of the produced steroids each haivng hydroxyl group at the 25-position was determined. The results are shown in Tables 4 and 5.

The cyclodextrins (CD) and derivatives thereof used were as follows:
α-CD, β-CD, γ-CD, hydroxypropyl-β-CD and maltosyl-β-CD.
PMCD-a: partially methylated cyclodextrin (methylation rate: 72.1 %)
PMCD-b: partially methylated cyclodextrin (methylation rate: 69.0 %)
PMCD-c: partially methylated cyclodextrin (methylation rate: 55.8 %)
Mixture of PMCD-1 : PMCD-a/PMCD-c = 1/2 mixture (methylation rate: 61.2 %)
Mixture of PMCD-2 : PMCD-a/PMCD-c = 2/1 mixture (methylation rate: 66.7 %)
DMCD: 2,6-di-O-methyl-β-cyclodextrin (methylation rate: 66.6 %)
TMCD: 2,3,6-tri-O-methyl-β-cyclodextrin (methylation rate: 100 %)
Mixture of PMCD-3 : DMCD/TMCD = 1/2 mixture (methylation rate: 88.9 %)
Mixture of PMCD-4 : PMCD/PMCD = 2/1 mixture (methylation rate: 77.7 %)

**Table 4**

| [Quantity of 25-hydroxylated steroid after 6 hour or 24 hour reaction] (unit: µg/ml) | | | | |
|---|---|---|---|---|
| | Reaction for 6 hours | | Reaction for 24 hours | |
| Tween 80 | none | 0.2 % | none | 0.2 % |
| Without CD | 10.7 | 11.3 | 8.76 | 9.38 |
| α-CD | 11.1 | 8.37 | 2.20 | 3.67 |
| β-CD | 7.53 | 20.0 | 14.3 | 13.0 |
| γ-CD | 13.7 | 34.7 | 17.2 | 9.97 |
| Hydroxypropyl-β-CD | 7.53 | 20.8 | 45.0 | 73.7 |
| Maltosyl-β-CD | 11.1 | 72.4 | 68.4 | 110 |
| PMCD-c | 107 | 88.4 | 160 | 157 |

The effects of all the cyclodextrins (excluding α-cyclodextrin) were observed. The effect of PMCD-c was the most remarkable, and the effects of other cyclodextrins were also improved by the combination with Tween 80 (TM) (reaction time: 6 hours). When Tween 80 (TM) was used alone, the effect was only slight.

**Table 5**

| [Quantity of 25-hydroxylated steroid (unit: µg/ml)] | | | |
|---|---|---|---|
| | Methylation rate (%) | Reaction time | |
| | | 6 hous | 24 hour |
| No CD | | 15.3 | 10.7 |
| PMCD-a | 72.1 | 71.4 | 130 |
| PMCD-b | 69.0 | 79.6 | 138 |
| PMCD-c | 55.8 | 89.4 | 144 |
| Mixed PMCD-1 | 61.2 | 115 | 180 |
| Mixed PMCD-2 | 66.7 | 87.7 | 149 |
| DMCD | 66.6 | 54.3 | 147 |
| TMCD | 100 | 43.4 | 108 |
| Mixed PMCD-3 | 88.9 | 50.4 | 126 |
| Mixed PMCD-4 | 77.7 | 44.9 | 128 |

It is apparent from the above results that all the methylated cyclodextrins were effective. In particular, the highest average methylation rate of 61 % was obtained when each of the methylated cyclodextrins was used.

### Example 5

The reaction was conducted using steroid a as the substrate for 6 hours or 24 hours in the same manner as in Example 1 except that various microorganisms were used in the absence or presence of 1 % PMCD, and the quantity of the steroid having hydroxyl group at the 25-position was determined. The results are shown in Table 6.

The microoorganisms used were as follows:
Amycolata saturnea A-1246 (FERM BP 5544)
Amycolata autotrophica (ATCC 33796)
Streptomyces roseosprous (FERM BP 1574)
Sphingomonas parapaucimobilis (IFO 15100)
Streptomyces sp. HB-103 (FERM BP 4318)

**Table 6**

| [Quantity of 25-hydroxylated steroid (unit: µg/ml)] | | | | |
|---|---|---|---|---|
| | 6 Hour reaction | | 24 hour reaction | |
| | No CD | 1% PMCD | No CD | 1% PMCD |
| Amycolata saturnea A-1246 | 10.7 | 107 | 8.76 | 160 |
| Amycolata autotrophica ATCC 33796 | 9.31 | 111 | 0 | 177 |
| Streptomyses roseosprous FERM BP 1574 | 2.65 | 4.29 | 0 | 10.4 |
| Sphingomonas prapaucimobilis IFO 15100 | 2.28 | 2.04 | 1.72 | 1.71 |
| Streptomyces sp. HB-103 | 5.50 | 30.5 | 5.21 | 90.5 |

### Exmple 6

100 ml of medium A was fed into a 500 ml Erlenmeyer flask. After stopping with a cotton stopper, it was sterilized with steam at 121 °C for 20 minutes. After cooling, one platinum wire loop of Amycolata autotrophica ATCC 33796 was inoculated thereinto, and the shaking culture was conducted at 28°C at 210 rpm for three days. 50 ml of medium B which further contained 1.0 % of PMCD (methylation rate: 55.8 %) was fed into each of four 250 ml Erlenmeyer flasks, the flasks were each stopped with a cotton stopper and sterilized at 121°C under a high pressure for 20 minutes. 1.0 ml of the culture fluid obtained with medium A as described above was poured into each flask under sterile conditions and then cultured at 28 °C at 220 rpm (amplitude: 70 mm) for two days. As the control, the same procedure as that described above was repeated except that PMCD-free medium B was used. A solution of 25 mg of steroid a in 1 ml of ethanol was added to each culture fluid and the culture was continued under the above-described conditions for 24 hours. A solution of 25 mg of steroid a in 1 ml of ethanol was added to the culture fluid and the culture was continued under the above-described conditions for 24 hours (concentration of steroid a added: 1 g/ℓ). After the completion of the reaction, 1 ml of the obtained culture fluid was fed into a centrifugal precipitation tube having a ground stopper. 9 ml of methanol was added to the fluid, the bottle was tightly stopped and they were mixed for 15 minutes. The liquid mixture was centrifuged at 3,000 rpm for 10 minutes, and the supernatant liquid was analyzed by HPLC. The analysis was conducted by using an HPLC apparatus of L-6000 System (a product of Hitachi, Ltd.), a column of YMC A 503CN (inner diameter: 4.6 mm, the whole length: 250 mm; a product of Yamamura Chemistry) and an eluent comprising acetonitrile and water (55:45) at a flow rate of 1.0 ml/min and at a column temperature of 40 °C . The detection was conducted according to the UV absorption at 205 or 265 nm. The quantitative analysis was conducted by the comparison with the areametric values of chemically synthesized steroid a hydrolyzed at the 25 position. As a result, the formation of 780 mg/l of steroid a hydroxylated at the 25-position was recognized. When PMCD was not added to the medium, the amount of steroid a hydroxylated at the 25-position was 9.1 mg/l.

200 ml of the reaction fluid obtained in the PMCD-containing medium B was centrifuged at 3,000 rpm for 10 minutes to obtain a supernatant liquid, which was passed through an Amberlite XAD-7 column (inner diameter: 30 mm, length: 140 mm; a product of Organo) to adsorb steroid a and also steroid a hydroxylated at the 25-position. After washing the column with water and 50 % methanol followed by the elution with 100 % methanol, the effluent was concentrated to dryness under reduced pressure.

The dry concentrate thus obtained was dissolved in a small amount of n-hexane / ethyl acetate (3:1) and the solution was adsorbed at the top of a column filled with 50 g of Wako Gel C-300 (TM) (a product of Wako Pure Chemical Industries, Ltd.) and n-hexane / ethyl acetate (3:1). 500 ml of n-hexane / ethyl acetate (3:1) was passed through the column. After the elution with n-hexane / ethyl acetate (3:2), the effluent fraction was analyzed by HPLC, and a fraction of steroid a hydroxylated at the 25-position was concentrated to dryness under reduced pressure.

Then the dry product was separated by HPLC under the following conditions: column: Inertsil Prep-ODS (TM) (20 × 250 mm; a product of GL Science Co., Ltd.), eluent: 90 % methanol, flow rate: 10 ml/min, and detection: UV absorption at 265 nm. As a result, 97 mg of steroid a hydroxylated at the 25-position was obtained. The structure of the isolated product was analyzed according to the nuclear magnetic resonance, infrared absorption and mass spectrum to confirm that it was the steroid a hydroxylated at the 25-position.

The properties of the obtained product hydroxylated at the 25-position were as follows:
m.p. : 169 to 171 °C
[α] _{D} : -16.0 (c 0.5, MeOH, 21.8 °C)
FAB-MS(M/Z):
   414(M+H)⁺ (Matrix: m-nitrobenzyl alcohol)
   (calculated: 413: C₂₇H₄₁O₃)
IR νₘₐₓ cm⁻¹:
   3382, 2959, 2870, 1449, 1377, 1267, 1151, 1047, 930
UV λₘₐₓ nm (ε):
   260(8860 sh), 269(12300), 280(12750), 291(7210)
¹H-NMR(CDCl₃) δ (ppm) :
   5.72(1H, d, J=4Hz), 5.40(1H, m), 3.91(1H, m), 3.33(1H, d, J=4Hz), 3.05(1H, d, J=4Hz), 2.4 to 2.5(2H, m), 2.24(1H, t, J=11Hz), 2.1 to 2.15(1H, m), 2.01(1H, d, J=5Hz), 1.88 to 1.95(2H, m), 1.78 to 1.85(2H, m), 1.66 to 1.71(1H, m), 1.22 to 1.5(11H, m), 1.22(6H, s), 1.05(3H, s), 0.97(3H, d, J=7Hz), 0.64(3H, s)
¹³C-NMR(CDCl₃) δ (ppm) :
   141.5(s), 133.7(s), 122.0(d), 115.9(d), 71.1(s), 67.2(d), 60.9(d), 60.2(d), 55.7(d), 54.5(d), 44.4(t), 42.7(s), 39.7(d), 38.8(t), 38.4(s), 36.9(t), 36.4(t), 36.1(d), 29.4(q), 29.2(q), 28.0(t), 23.0(t), 20.8(t), 20.6(t), 18.8(q), 15.2(q), 11.9(q)

### Example 7

The reaction was conducted for six hours in the same manner as that of Example 2, and the steroids having hydroxyl group at the 25-position were determined. The results are shown in Table 7.

**Table 7**

| [Quantity of 25-hydroxylated steroid after 6 hour reaction] | | | | | |
|---|---|---|---|---|---|
| PMCD concentration (%) | 0 | 0.25 | 0.5 | 1.0 | 2.0 |
| 25-OH-steroid (µg/ml) | 4.0 | 7.5 | 24 | 8.0 | 7.5 |

It is apparent from the above-described results that the optimum concentration of the partially methylated cyclodextrin is 0.5 %.

### Example 8

The reaction was conducted using steroid b as the substrate for six hours in the same manner as that of Example 3. The quantity of the steroid having hydroxyl group at the 25-position was determined. The results are shown in Table 8.

**Table 8**

| [Quantity of 25-hydroxylated steroid after 6 hour reaction] | | | | | |
|---|---|---|---|---|---|
| Substrate conc. (%) | 65 | 125 | 250 | 500 | 1000 |
| 25-OH-steroid (µg/ml) | 10.5 | 17.4 | 23.2 | 8.50 | 3.45 |

It is apparent from the above results that the optimum concentration of the steroid is 250 µg/ml.

### Example 9 Synthesis of steroid b hydroxylated at the 25-position:

200 ml of a culture fluid (to be divided and fed into four flasks) was obtained by culturing Amycolata autotrophica ATCC 33796 in PMCD-containing culture B in the same manner as that of Example 6. A solution of 25 mg of steroid b in 1 ml of ethanol was added to the culture fluid in each flask and the culture was continued under the above-described conditions for 24 hours. A solution of 25 mg of steroid b in 1 ml of ethanol was added to the culture fluid and the culture was continued under the above-described conditions for 24 hours (concentration of steroid b added: 1 g/ℓ). As a result, the formation of 778 mg/l of steroid b hydroxylated at the 25-position was accumulated in the PMCD-containing medium B. When PMCD was not added to the medium, the amount of steroid b hydroxylated at the 25-position was 4.0 mg/l.

200 ml of the reaction fluid obtained in the PMCD-containing medium B was centrifuged at 3,000 rpm for 10 minutes to obtain a supernatant liquid, which was passed through an Amberlite XAD-7 column (inner diameter: 30 mm, length: 140 mm; a product of Organo) to adsorb steroid b and also steroid b hydroxylated at the 25-position. After washing the column with water and then with 50 % methanol followed by teh elution with 100 % methanol, the effluent was concentrated to dryness under reduced pressure.

The dry concentrate thus obtained was dissolved in a small amount of dichloromethane / ethanol (19:1) and the solution was adsorbed at the top of a column filled with 50 g of Wako Gel C-300 (TM) (a product of Wako Pure Chemical Industries, Ltd.) and dichloromethane /ethanol (19:1). After the elution with dichloromethane/ethanol (19:1), the effluent fraction was analyzed by HPLC, and a fraction of steroid b hydroxylated at the 25-position was concentrated to dryness under reduced pressure to obtain 103 mg of steroid b hydroxylated at the 25-position.
m.p. : 155 to 157°C (crystallized from chloroform)
[α] _{D} : 44.6 (c 0.5, MeOH, 22 °C)
FAB-MS(m/z):
490(M)⁺ (matrix: m-nitrobenzyl alcohol, Positive mode)
489(M-H) (matrix: m-nitrobenzyl alcohol Native mode
(Calculated:490 C₃₀H₅₀O₅)
IRνₘₐₓ cm⁻¹ :
3385, 2938, 2870, 1468, 1379, 1136, 1096, 1055, 910
UVλ ₘₐₓ nm (ε) :
262(6570 sh), 272(9400), 282(10010), 294(5850)
¹H-NMR(CDCl₃) δ (ppm) :
5.71 (1H, m, J=5.5, 2.2Hz), 5.40 (1H, m, J=5.5, 2.6Hz), 3.6 to 4.0(7H, m), 2.5 to 2.7 (2H, m), 2.32 (1H, dd, J=14, 5Hz), 2.10 (1H, m), 1.23 to 2.0 (22H, m), 1.22 (6H, s), 1.07 (3H, s), 0.96 (3H, d, J=6.6Hz), 0.63 (3H,s)
¹³C-NMR(CDCl₃)δ (ppm) :
141.0(s), 136.1(s), 124.6(d), 115.4(d), 82.3(d), 71.8(d), 71.1(s), 68.9(t), 67.0(d), 60.8(t), 55.9(d), 54.6(d), 44.4(t), 43.1(s),41.8(s), 39.2(t), 38.7(d), 36.4(t), 36.1(d), 35.2(t), 32.3(t), 29.4(q),29.2(q), 28.1(t), 23.0(t), 21.1(t), 20.8(t), 18.8(q), 15.9(q), 11.9(q)

## Claims

1. A biological process for producing steroids hydroxylated at the 25-position thereof, which comprises adding a steroid (excluding cholesterol) to the cells or culture liquid of a microorganism of the genus *Amycolata* or *Sphingomonas* capable of hydroxylating the steroid at the 25-position thereof to convert a hydrogen atom bonded to a carbon atom at the 25-position of the steroid into a hydroxyl group, the process **characterised in that** the steroid has the following general formula (I): 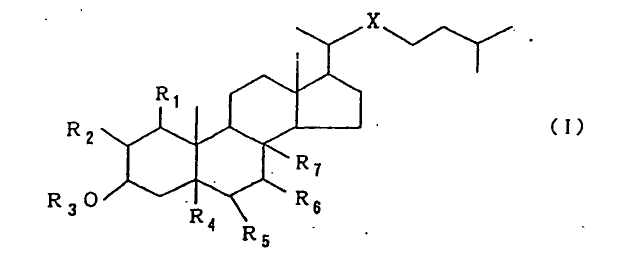 wherein R₁ represents a hydrogen atom or a hydroxyl group which may be optionally protected; R₂ represents a hydrogen atom or a hydroxy-lower alkoxy group in which the hydroxyl group may be optionally protected, or R₁ and R₂ together form a double bond or epoxy ring; R₃ represents a hydrogen atom or a protecting group; R₄, R₅, R₆ and R₇ each represent a hydrogen atom, one or both couples of R₄ and R₅, and R₆ and R₇ form a double bond or, alternatively, R₅ and R₆ together form a double bond and R₄ and R₇ are bonded to a dienophile capable of protecting the conjugated double bond; and X represents CH₂ or an oxygen atom, with the proviso that when X is CH₂, a compound wherein R₁, R₂, R₆ and R₇ are hydrogen atom and R₄ and R₅ together form a double bond is excluded.

2. The process of claim 1 wherein the steroid is a compound selected from the group consisting of 1α,3β-dihydroxy-5,7-cholestadiene, 2β-(3-hydroxypropyloxy)-1α,3β-dihydroxy-5,7-cholestadiene, 5α,8α-(3,5-dioxo-4-phenyl-1,2,4-triazolizino)-1,6-cholestadiene-3β-ol, 3β-hydroxy-1,5,7-cholestatriene, 1α,2α-epoxy-5α,8α-(3,5-dioxo-4-phenyl-1,2,4-triazolizino)-6-cholesten-3β-ol,1α,2α-epoxy-3β hydroxy-5,7-cholestadiene, and 20(S)-(3-methylbutyloxy)-pregna-5-ene-1 α,3β-diol.

3. The process of claim 2, wherein the steroid is 1α,3β-dihydroxy-5,7-cholestadiene.

4. The process of claim 2, wherein the steroid is 2β-(3-hydroxypropyloxy)-1α,3β-dihydroxy-5,7-cholestadiene.

5. The process of claim 2, wherein the steroid is 5α,8α-(3,5-dioxo-4-phenyl-1,2,4-triazolizino)-1,6-cholestadiene-3β-ol.

6. The process of claim 2, wherein the steroid is 3β-hydroxy-1,5,7-cholestatriene.

7. The process of claim 2, wherein the steroid is 1α,2α-epoxy-5α,8α -(3,5-dioxo-4-phenyl-1,2,4-triazolizino)-6-cholesten-3β-ol.

8. The process of claim 2, wherein the steroid is 1α,2α-epoxy-3β-hydroxy-5,7-cholestadiene.

9. The process of claim 2, wherein the steroid is 20(S)-(3-methylbutyloxy)-pregna-5-ene-1α,3β-diol.

10. The process of claim 1, wherein the microorganisms of the genus Amycolata are Amycolata saturnea.

11. The process of claim 1, wherein the microorganisms of the genus Amycolata are Amycolata saturnea FERM BP 5544.

12. The process of claim 1, wherein the microorganisms of the genus Amycolata are Amycolata autotrophica.

13. The process of claim 1, wherein the microorganisms of the genus Amycolata are Amycolata autotrophica ATCC 33796.

14. The process of claim 1, wherein the microorganisms of the genus Sphingomonas are Sphingomonas parapaucimobilis.

15. The process of claim 1, wherein the microorganisms of the genus Sphingomonas are Sphingomonas parapaucimobilis IFO 15100.

16. The process of any of claims 1 to 15, which is conducted in the presence of a cyclodextrin or a cyclodextrin derivative.

17. The process of claim 16, wherein the cyclodextrin is β- or γ-cyclodextrin.

18. The process of claim 16, wherein the cyclodextrin derivative is a methylated cyclodextrin.

19. The process of claim 18, wherein the methylation rate of the methylated cyclodextrin is 50 to 70 %.

20. The process of claim 18, wherein the methylation rate of the methylated cyclodextrin is 61 %.

21. The process of any of claims 16 to 20, wherein the amount of the cyclodextrin or the cyclodextrin derivative is 0.1 to 10 mg per ml of the reaction solution.

22. The process of claim 21, wherein the amount of the cyclodextrin or the cyclodextrin derivative is 0.5 to 5 mg per ml of the reaction solution.

23. The process of any of claims 16 to 22, wherein the reaction is conducted in the presence of a surfactant.

24. The process of claim 23, wherein the surfactant is a nonionic surfactant.

25. The process of claim 24, wherein the nonionic surfactant is at least one surfactant selected from the group consisting of polyoxyethylene / sorbitan fatty acid esters, sorbitan / fatty acid esters, polyoxyethylene ethers, Triton X-100, nonylphenol and ethylene oxide / propylene oxide block copolymer s.

26. The process of claim 24 or 25, wherein the nonionic surfactant is used in an amount of 0.1 to 0.5 %.

## Patentansprüche

1. Ein biologisches Verfahren zur Herstellung von Steroiden, die in ihrer 25-Position hydroxyliert sind, umfassend die Zugabe eines Steroids (ausgenommen Cholesterin) zu den Zellen oder der Kulturflüssigkeit eines Mikroorganismus des Gattung *Amycolata* oder *Sphingomonas,* der fähig ist, dessen Steroid in der 25-Position zu hydroxylieren, um ein Wasserstoffatom, das an ein Kohlenstoffatom an der 25-Postion des Steroids gebunden ist, in eine Hydroxylgruppe umzuwandeln, wobei das Verfahren **dadurch gekennzeichnet ist, dass** das Steroid die folgende allgemeine Formel (I) aufweist: 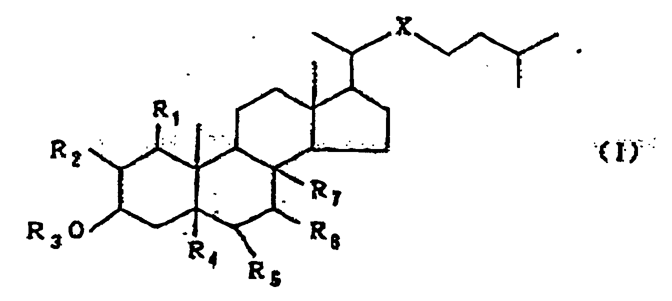 wobei R₁ ein Wasserstoffatom oder eine Hydroxylgruppe, die optional geschützt sein kann, darstellt; R₂ ein Wasserstoffatom oder eine Hydroxyl-substituierte, niedere Alkoxygruppe, in der die Hydroxyl-Gruppe optional geschützt sein kann, darstellt, oder R₁ und R₂ zusammen eine Doppelbindung oder einen Epoxidring bilden; R₃ ein Wasserstoffatom oder eine Schutzgruppe darstellt; R₄, R₅, R₆ und R₇ jeweils ein Wasserstoffatom darstellen, eines oder beide der Paare aus R₄ und R₅ bzw. R₆ und R₇ eine Doppelbindung bilden oder, alternativ, R₅ und R₆ zusammen eine Doppelbindung bilden und R₄ und R₇ an ein Dienophil gebunden sind, das fähig ist, die konjugierte Doppelbindung zu schützen; und X CH₂ oder ein Sauerstoffatom darstellt, unter der Bedingung, dass wenn X CH₂ ist, eine Verbindung, in der R₁, R₂, R₆ und R₇ Wasserstoffatome sind und R₄ und R₅ zusammen eine Doppelbindung bilden, ausgeschlossen ist.

2. Verfahren gemäß Anspruch 1, wobei das Steroid eine Verbindung ist, die ausgesucht ist aus der Gruppe bestehend aus 1α,3β-Dihydroxy-5,7-cholestadien, 2β*-*(3-Hydroxypropyloxy)-1α,3β-dihydroxy-5,7-cholestadien, 5α,8α-(3,5-Dioxo-4-phenyl-1,2,4-triazolizino)-1,6-cholestadien-3β-ol, 3β-Hydroxy-1,5,7-cholestatrien, 1α,2α-Epoxy-5α,8α-(3,5-dioxo-4-phenyl-1,2,4-triazolizino)-5-cholesten-3β-ol, 1α,2α-Epoxy-3β-hydroxy-5,7-cholestadien und 20(S)-(3-Methylbutyloxy)-pregna-5-en-1α,3β-diol.

3. Verfahren gemäß Anspruch 2, wobei das Steroid 1α,3β-Dihydroxy-5,7-cholestadien ist.

4. Verfahren gemäß Anspruch 2, wobei das Steroid 2β-(3-Hydroxypropyloxy)-1α,3β dihydroxy-5,7-cholestadien ist.

5. Verfahren gemäß Anspruch 2, wobei das Steroid 5α,8α-(3,5-Dioxo-4-phenyl-1,2,4-triazolizino)-1,6-cholestadien-3β-ol ist.

6. Verfahren gemäß Anspruch 2, wobei das Steroid 3β-Hydroxy-1,5,7-cholestatrien ist.

7. Verfahren gemäß Anspruch 2, wobei das Steroid 1α,2α-Epoxy-5α,8α-(3,5-dioxo-4-phenyl-1,2,4-triazolizino)-6-cholesten-3β-ol ist.

8. Verfahren gemäß Anspruch 2, wobei das Steroid 1*α,*2α-Epoxy-3β-hydroxy-5,7-cholestadien ist.

9. Verfahren gemäß Anspruch 2, wobei das Steroid 20(S)-(3-Methylbutyloxy)-pregna-5-en-1α,3β-diol ist.

10. Verfahren gemäß Anspruch 1, wobei die Mikroorganismen vom Gattung Amycolata Amycolata saturnea sind.

11. Verfahren gemäß Anspruch 1, wobei die Mikroorganismen vom Gattung Amycolata Amycolata saturnea FERM BP 5544 sind.

12. Verfahren gemäß Anspruch 1, wobei die Mikroorganismen vom. Gattung Amycolata Amycolata autotrophica sind.

13. Verfahren gemäß Anspruch 1, wobei die Mikroorganismen vom Gattung Amycolata Amycolata autotrophica ATCC 33796 sind.

14. Verfahren gemäß Anspruch 1, wobei die Mikroorganismen vom Gattung Sphingomonas Sphingomonas parapaucimobilis sind.

15. Verfahren gemäß Anspruch 1, wobei die Mikroorganismen vom, Gattung Sphingomonas Sphinigomonas parapaucimobilis IFO 15100 sind.

16. Verfahren gemäß einem der Ansprüche 1 bis 15, das in der Gegenwart eines Cyclodextrins oder Cyclodextrin-Derivates durchgeführt wird.

17. Verfahren gemäß Anspruch 16, wobei das Cyclodextrin *β-* oder γ-Cyclodextrin ist.

18. Verfahren gemäß Anspruch 16, wobei das Cyclodextrin-Derivat ein methyliertes Cyclodextrin ist.

19. Verfahren gemäß Anspruch 18, wobei der Methylierungsgrad des methylierten Cyclodextrins von 50 bis 70% beträgt.

20. Verfahren gemäß Anspruch 18, wobei der Methylierungsgrad des methylierten Cyclodextrins 61 % beträgt.

21. Verfahren gemäß einem der Ansprüche 16 bis 20, wobei die Menge des Cyclodextrins oder des Cyclodextrinderivates von 0.1 bis 10 mg pro ml der Reaktionslösung beträgt.

22. Verfahren gemäß Anspruch 21, wobei die Menge des Cyclodextrins oder des Cyclodextrinderivates von 0,5 bis 5 mg pro ml der Reaktionslösung beträgt.

23. Verfahren gemäß einem der Ansprüche 16 bis 22, wobei die Reaktion in Gegenwart einer oberflächenaktiven Substanz durchgeführt wird.

24. Verfahren gemäß Anspruch 23, wobei die oberflächenaktive Substanz eine nichtionische oberflächenaktive Substanz ist.

25. Verfahren gemäß Anspruch 24, wobei die nichtionische oberflächenaktive Substanz zumindest eine oberflächenaktive Substanz ist, die ausgesucht ist aus der Gruppe bestehend aus Polyoxyethylen / Sorbitan-Fettsäureester, Sorbitan / Fettsäureester, Polyoxyethylenether, Triton X-100, Nonylphenol und Ethylenoxid / Propylenoxid-Blockcopolymere.

26. Verfahren gemäß Anspruch 24 oder 25, wobei die nichtionische oberflächenaktive Substanz in einer Menge von 0.1 bis 0,5% verwendet wird.

## Revendications

1. Procédé biologique pour produire des stéroïdes hydroxylés en position 25, qui comprend le fait d'ajouter un stéroïde (à l'exclusion du cholestérol) à des cellules ou un liquide de culture d'un microorganisme du genre *Amycolata* ou *Sphingomonas,* capable d'hydroxyler le stéroïde en position 25 pour remplacer un atome d'hydrogène lié à un atome de carbone en position 25 du stéroïde par un groupe hydroxyle, procédé **caractérisé en ce que** le stéroïde a la formule générale suivante (I) : 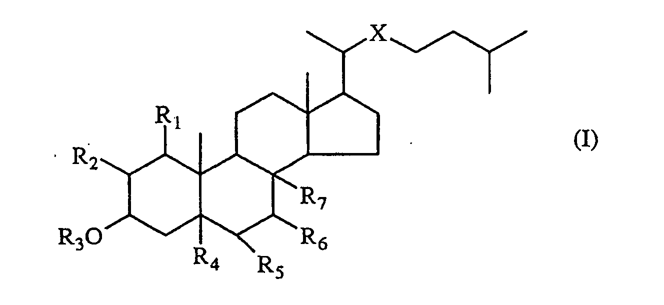 dans laquelle R₁ représente un atome d'hydrogène ou un groupe hydroxyle qui peut être protégé de façon optionnelle, R₂ représente un atome d'hydrogène ou un groupe hydroxyalcoxy inférieur dont le groupe hydroxyle peut être protégé de façon optionnelle, ou R₁ et R₂ représentent ensemble une double liaison ou un cycle époxy, R₃ représente un atome d'hydrogène ou un groupe protecteur, R₄, R₅, R₆ et R₇ représentent chacun un atome d'hydrogène, l'une des couples (R₄, R₅) et (R₆, R₇) ou les deux représentent une double liaison, ou bien R₅ et R₆ représentent ensemble une double liaison et R₄ et R₇ sont liés à un diénophile capable de protéger la double liaison conjuguée, et X représente CH₂ ou un atome d'oxygène, sous réserve que soit exclu, si X représente CH₂, le composé dans lequel R₁, R₂, R₆ et R₇ représentent des atomes d'hydrogène et R₄ et R₅ représentent ensemble une double liaison.

2. Procédé selon la revendication 1, dans lequel le stéroïde est un composé choisi dans l'ensemble constitué par les suivants : 1α,3β-dihydroxy-5,7-cholestadiène, 2β-(3-hydroxypropyloxy)-1α,3β-dihydroxy-5,7-cholestadiène, 5α,8α-(3,5-dioxo-4-phényl-1,2,4-triazolizino)-1,6-cholestadiène-3β-ol, 3β-hydroxy-1,5,7-cholestatriène, 1α,2α-époxy-5α,8α-(3,5-dioxo-4-phényl-1,2,4-triazolizino)-6-cholestèn-3β-ol, 1α,2α-époxy-3β-hydroxy-5,7-cholestadiène, et 20(S)-(3-méthylbutyloxy)-prégna-5-ène-1α,3β-diol.

3. Procédé selon la revendication 2, dans lequel le stéroïde est le 1α,3β-dihydroxy-5,7-cholestadiène.

4. Procédé selon la revendication 2, dans lequel le stéroïde est le 2β-(3-hydroxypropyloxy)-1α,3β-dihydroxy-5,7-cholestadiène.

5. Procédé selon la revendication 2, dans lequel le stéroïde est le 5α,8α-(3,5-dioxo-4-phényl-1,2,4-triazolizino)-1,6-cholestadiène-3β-ol.

6. Procédé selon la revendication 2, dans lequel le stéroïde est le 3β-hydroxy-1,5,7-cholestatriène.

7. Procédé selon la revendication 2, dans lequel le stéroïde est le 1α,2α-époxy-5α,8β-(3,5-dioxo-4-phényl-1,2,4-triazolizino)-6-cholestèn-3β-ol.

8. Procédé selon la revendication 2, dans lequel le stéroïde est le 1α,2α-époxy-3β-hydroxy-5,7-cholestadiène.

9. Procédé selon la revendication 2, dans lequel le stéroïde est le 20(S)-(3-méthylbutyloxy)-pregna-5-ène-1α,3β-diol.

10. Procédé selon la revendication 1, dans lequel les microorganismes du genre *Amycolata* sont des *Amycolata saturnea.*

11. Procédé selon la revendication 1, dans lequel les microorganismes du genre *Amycolata* sont des *Amycolata saturnea* FERM BP 5544.

12. Procédé selon la revendication 1, dans lequel les microorganismes du genre *Amycolata* sont des *Amycolata autotrophica.*

13. Procédé selon la revendication 1, dans lequel les microorganismes du genre *Amycolata* sont des *Amycolata autotrophica* ATCC 33796.

14. Procédé selon la revendication 1, dans lequel les microorganismes du genre *Sphingomonas* sont des *Sphingomonas parapaucimobilis.*

15. Procédé selon la revendication 1, dans lequel les microorganismes du genre *Sphingomonas* sont des *Sphingomonas parapaucimobilis* IFO 15100.

16. Procédé selon l'une quelconque des revendications 1 à 15, qui est mmis en oeuvre en présence d'une cyclodextrine ou d'un dérivé de cyclodextrine.

17. Procédé selon la revendication 16, dans lequel la cyclodextrine est une cyclodextrine β ou γ.

18. Procédé selon la revendication 16, dans lequel le dérivé de cyclodextrine est une cyclodextrine méthylée.

19. Procédé selon la revendication 18, dans lequel le taux de méthylation de la cyclodextrine méthylée vaut de 50 à 70 %.

20. Procédé selon la revendication 18, dans lequel le taux de méthylation de la cyclodextrine méthylée vaut 61 %.

21. Procédé selon l'une quelconque des revendications 16 à 20, dans lequel la quantité de cyclodextrine ou de dérivé de cyclodextrine vaut de 0,1 à 10 mg par ml de solution de réaction.

22. Procédé selon la revendication 21, dans lequel la quantité de cyclodextrine ou le dérivé de cyclodextrine vaut de 0,5 à 5 mg par ml de solution de réaction.

23. Procédé selon l'une quelconque des revendications 16 à 22, dans lequel la réaction est effectuée en présence d'un surfactant.

24. Procédé selon la revendication 23, dans lequel le surfactant est un surfactant non-ionique.

25. Procédé selon la revendication 24, dans lequel le surfactant non-ionique est au moins un surfactant choisi dans le groupe constitué par les esters de sorbitane polyéthoxylé et d'acide gras, les esters de sorbitane et d'acide gras, les éthers de polyoxyéthvl'cne, le Triton X-100, le nonylphénol et les copolymères séquencés d'oxyde d'éthylène et d'oxyde de propylène.

26. Procédé selon l'une quelconque des revendications 24 et 25, dans lequel le surfactant non-ionique est utilisé en une quantité de 0,1 à 0,5 %.
